(19) **Europäisches Patentamt / European Patent Office / Office européen des brevets**

(11) **EP 3 319 723 B1**

(12) **FASCICULE DE BREVET EUROPEEN**

(45) Date de publication et mention de la délivrance du brevet:
**10.11.2021 Bulletin 2021/45**

(21) Numéro de dépôt: **16741569.4**

(22) Date de dépôt: **08.07.2016**

(51) Int Cl.:
*B01J 20/18* (2006.01)    *B01J 20/30* (2006.01)
*C07C 7/13* (2006.01)    *C07C 15/08* (2006.01)
*C07C 29/76* (2006.01)    *C07C 15/02* (2006.01)
*C07C 37/82* (2006.01)    *C07C 39/07* (2006.01)
*C07C 201/16* (2006.01)    *C07C 205/06* (2006.01)
*C07C 209/86* (2006.01)    *C07C 211/50* (2006.01)

(86) Numéro de dépôt international:
**PCT/EP2016/066293**

(87) Numéro de publication internationale:
**WO 2017/005907 (12.01.2017 Gazette 2017/02)**

(54) **ADSORBANTS ZÉOLITHIQUES, LEUR PROCÉDÉ DE PRÉPARATION ET LEURS UTILISATIONS**

ZEOLITHISCHE ADSORPTIONSMITTEL, VERFAHREN ZUR HERSTELLUNG DAVON UND VERWENDUNGEN DAVON

ZEOLITIC ADSORBENTS, METHOD FOR THE PRODUCTION THEREOF, AND USES OF SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.07.2015 FR 1556523**

(43) Date de publication de la demande:
**16.05.2018 Bulletin 2018/20**

(73) Titulaires:
• **IFP Energies nouvelles**
**92500 Rueil-Malmaison (FR)**
• **ARKEMA FRANCE**
**92700 Colombes (FR)**

(72) Inventeurs:
• **LAROCHE, Catherine**
**69390 Vernaison (FR)**
• **BOUVIER, Ludivine**
**64300 Orthez (FR)**
• **PEREZ-PELLITERO, Javier**
**69007 Lyon (FR)**

(74) Mandataire: **Bandpay & Greuter**
**30, rue Notre-Dame des Victoires**
**75002 Paris (FR)**

(56) Documents cités:
FR-A1- 2 789 914    FR-A1- 2 903 978
US-A- 3 663 638    US-A- 3 686 342

EP 3 319 723 B1

**Description**

DOMAINE TECHNIQUE

L'invention concerne des adsorbants à base de cristaux agglomérés de zéolithe X comprenant du baryum et du potassium, leur procédé de préparation et leurs utilisations.

Ces adsorbants peuvent être utilisés plus particulièrement pour la production en phase liquide ou phase gaz de para-xylène très pur à partir d'une charge d'hydrocarbures aromatiques contenant des isomères à 8 atomes de carbone.

TECHNIQUE ANTERIEURE

Il est connu dans l'art antérieur que des adsorbants comprenant des aluminosilicates cristallins peuvent être utilisés pour séparer certains hydrocarbures à partir de mélanges les contenant. Dans le domaine de la séparation d'hydrocarbures aromatiques et en particulier la séparation d'isomères en C8 aromatiques, il est généralement reconnu que l'utilisation de cations particuliers dans les sites cationiques d'aluminosilicates cristallins zéolithiques améliore la sélectivité de la zéolithe pour un des isomères en C8-aromatique. Cette adsorption différenciée au sein de la zéolithe permet la séparation des différents isomères en C8-aromatique, ce qui est utilisé industriellement pour la production de para-xylène très pur à partir d'une charge d'hydrocarbures aromatiques contenant des isomères à 8 atomes de carbone.

Ainsi, l'utilisation d'adsorbants zéolithiques constitués de zéolithes X ou Y comprenant, outre des cations sodium, des ions baryum, potassium ou strontium, seuls ou en mélanges, pour adsorber sélectivement en phase liquide le para-xylène dans un mélange d'hydrocarbures aromatiques, est bien connue de l'art antérieur.

Les brevets US 3 558 730, US 3 558 732, US 3 626 020, US 3 663 638 et US 3 960 774 montrent que des adsorbants zéolithiques comprenant des aluminosilicates de structure faujasite (FAU) à base de sodium et de baryum ou à base de sodium, de baryum et de potassium, sont efficaces pour la séparation du *para*-xylène présent dans des coupes aromatiques en C8 (coupes comprenant des hydrocarbures aromatiques à 8 atomes de carbone). Les adsorbants ci-dessus sont de préférence utilisés comme agents d'adsorption dans les procédés en phase liquide, notamment de type contre-courant simulé, similaires à ceux décrits dans le brevet US 2 985 589 et qui s'appliquent, entre autres, aux coupes aromatiques en C8.

Cependant, de manière générale, les propriétés d'adsorption des zéolithes pour les hydrocarbures aromatiques à 8 atomes de carbone (xylènes et éthylbenzène) varient de manière très fine en fonction de la taille et de la forme des pores ainsi que de la position des cations à l'intérieur de la structure qui influent à la fois sur le champ électrostatique présent à l'intérieur de la zéolithe et sur la forme du volume accessible dans les pores. D'autres paramètres, tels que la polarisabilité des cations et des molécules ou la flexibilité de la structure peuvent également avoir une influence. Il est donc extrêmement difficile de prévoir théoriquement et avec précision les caractéristiques d'adsorption d'une zéolithe vis-à-vis des hydrocarbures aromatiques à 8 atomes de carbone.

Pour améliorer la sélectivité d'adsorption de zéolithes ayant la structure faujasite pour les isomères aromatiques en C8, de nombreuses études ont fait mention de l'influence du rapport Si/Al de la zéolithe, de la nature des cations d'échange, ainsi que de leur teneur en eau. De la même manière, il est très difficile de prédire le degré d'amélioration parce que ces facteurs exercent des actions combinées sur les caractéristiques d'adsorption des zéolithes. En particulier, il est difficile de prévoir l'impact de la proportion relative d'ions baryum, et potassium dans le cas d'une zéolithe de structure faujasite (FAU) à base de baryum et de potassium, et plus précisément dans le cas d'une zéolithe de structure faujasite (FAU) de type X, à base de baryum et de potassium.

Le brevet FR 2 903 978 enseigne que les ions potassiums peuvent représenter jusqu'à 1/3 des sites échangeables occupés par le baryum et le potassium, mais ce brevet n'exemplifie aucun adsorbant contenant du potassium et ne fournit aucun enseignement permettant d'anticiper l'impact du potassium sur les sélectivités d'adsorption.

Les brevets US 8,283,274 et US 8,557,028 décrivent des adsorbants présentant des teneurs en poids en potassium comprises entre 0,25% et 0,9% en poids, correspondant à des rapports molaires $K_2O/(BaO+K_2O+Na_2O)$ compris entre 1,3% et 4,5%. Le brevet US 8,557,028 revendique des adsorbants ayant des teneurs en poids en potassium comprises entre 0,9 et 1,5%, équivalents à des rapports molaires $K_2O/(BaO+K_2O+Na_2O)$ compris entre 4,5% et 7,5%. Les exemples de ce dernier brevet montrent que la productivité est améliorée avec des adsorbants présentant des teneurs en potassium comprises entre 0,5% et 0,7% en poids, équivalents à des rapports molaires $K_2O/(BaO+K_2O+Na_2O)$ de 2,5% et 3,5%. Le brevet CN1267185 décrit des adsorbants présentant des rapports molaires $BaO/K_2O$ compris entre 10 et 40 correspondant à des rapports molaires $K_2O/(BaO+K_2O+Na_2O)$ compris entre 2,4% et 9,1%.

Le brevet récent US 2015/0105600 décrit quant à lui un adsorbant à base de zéolithe X, de baryum et de potassium, ayant un rapport molaire $K_2O/(BaO+K_2O+Na_2O)$ compris entre 15% et 40%.

La synthèse des zéolithes conduit à des cristaux (généralement sous forme de poudre) dont l'emploi à l'échelle industrielle est particulièrement malaisé (pertes de charges importantes lors des manipulations). On préfère alors les formes agglomérées de ces cristaux, sous forme de grains, de filés et autres agglomérés, ces dites formes pouvant être obtenues par extrusion, pastillage, et autres techniques d'agglomération connues de l'homme du métier. Ces agglomérés ne présentent pas les inconvénients inhérents aux matières pulvérulentes.

Ces agglomérés, qu'ils soient sous forme de plaquettes, de billes, d'extrudés, et autres, sont en général formés de cristaux de zéolithe(s), qui constituent l'élément actif (au sens de l'adsorption) et d'un liant destiné à assurer la cohésion des cristaux sous forme d'agglomérés et de leur conférer une résistance mécanique suffisante pour résister aux vibrations et aux mouvements auxquels ils sont soumis au cours des opérations de séparations des isomères des coupes aromatiques en C8. Cependant, les propriétés d'adsorption de ces agglomérés sont évidemment réduites par rapport à la poudre de cristaux, en raison de la présence de liant d'agglomération inerte vis-à-vis de l'adsorption. Divers moyens ont déjà été proposés pour pallier cet inconvénient du liant d'agglomération d'être inerte quant aux performances d'adsorption, parmi lesquels, la transformation de la totalité ou d'au moins une partie du liant d'agglomération en zéolithe active du point de vue de l'adsorption. Cette opération est maintenant bien connue de l'homme du métier, par exemple sous la dénomination de « zéolithisation ». Pour effectuer facilement cette opération, on utilise des liants zéolithisables, le plus souvent des argiles appartenant à la famille de la kaolinite, et de préférence préalablement calcinés à des températures généralement comprises entre 500°C et 700°C.

La demande de brevet FR 2 789 914 décrit par exemple un procédé de fabrication d'agglomérés de zéolithe X, de rapport Si/Al compris entre 1,15 et 1,5, contenant du baryum et éventuellement du potassium. Les agglomérés ainsi obtenus, après zéolithisation du liant, présentent, du point de vue de l'adsorption du para-xylène contenu dans les coupes aromatiques en C8, des propriétés améliorées par rapport à des adsorbants préparés à partir de la même quantité de zéolithe X et de liant, mais dont le liant n'est pas zéolithisé.

Les facteurs importants qui influencent les performances d'un procédé de séparation par adsorption englobent notamment la sélectivité d'adsorption, la capacité d'adsorption et la cinétique de transfert de matière qui définit les vitesses d'adsorption et de désorption des différents composés. L'adsorbant doit donc présenter de bonnes propriétés de transfert de matière afin de garantir un nombre de plateaux théoriques suffisants pour réaliser une séparation efficace des espèces en mélange, comme l'indique Ruthven dans l'ouvrage intitulé « Principles of Adsorption and Adsorption Processes » (« Principes de l'Adsorption et des Procédés d'Adsorption »), John Wiley & Sons, (1984), pages 326 et 407. Ruthven indique (ibid., page 243), que, dans le cas d'un adsorbant aggloméré, le transfert de matière global dépend de l'addition de la résistance diffusionnelle intra-cristalline et de la résistance diffusionnelle entre les cristaux. La résistance diffusionnelle intra-cristalline est proportionnelle au carré des rayons des cristaux et inversement proportionnelle à la diffusivité des molécules intracristalline.

La résistance diffusionnelle entre les cristaux (également appelée résistance macroporeuse) est quant à elle proportionnelle au carré des rayons des agglomérés et inversement proportionnelle à la diffusivité des molécules dans les macropores. Pour une structure zéolithique donnée, une taille d'aggloméré donnée et une température de fonctionnement donnée, les diffusivités sont fixées, et le seul moyen d'améliorer le transfert de matière consiste à réduire le diamètre des cristaux. Un gain sur le transfert global sera ainsi obtenu en réduisant la taille des cristaux.

Par conséquent, l'homme du métier s'attend à ce que des adsorbants zéolithiques agglomérés présentent à la fois une bonne capacité d'adsorption des xylènes et une bonne sélectivité pour le *para*-xylène, possèdent de très bonnes propriétés de séparation des xylènes lorsqu'ils sont réalisés à partir de petits cristaux de zéolithe dans les procédés en phase liquide de séparation du *para*-xylène contenu dans les coupes aromatiques en C8, par exemple de type contre-courant simulé. L'homme du métier est cependant dans l'impossibilité de définir a *priori* ou théoriquement et avec précision les caractéristiques d'adsorption d'une zéolithe FAU, notamment de type X, présentant une composition particulière en baryum et potassium, vis-à-vis des hydrocarbures aromatiques à 8 atomes de carbone.

La présente invention a pour objectif de fournir des nouveaux adsorbants à base de zéolithe X comprenant du baryum, du potassium et du sodium et présentant une composition particulière en baryum, potassium et sodium, optimale pour maximiser la productivité du procédé de séparation du *para*-xylène contenu dans les coupes aromatiques en C8. La présente invention propose également un procédé de séparation des xylènes mettant en oeuvre un adsorbant à base de zéolithe X présentant une composition particulière en baryum, potassium et sodium, permettant la production de para-xylène à haute pureté avec une productivité améliorée à partir d'une charge d'hydrocarbures aromatiques contenant des isomères à 8 atomes de carbone.

RESUME DE L'INVENTION

La présente invention est définie par les revendications annexées. L'invention concerne un adsorbant zéolithique comprenant des cristaux de zéolithe X et comprenant du baryum, du potassium et du sodium, dans lequel la teneur en oxyde de sodium Na2O est inférieure à 0,3% en poids par rapport à la masse totale de l'adsorbant et dans lequel le ratio molaire $K_2O / (K_2O + BaO + Na_2O)$ (des espèces sous forme d'oxydes) est compris entre 8,0% et 8,6%, de préférence entre 8,2% et 8,4%, l'incertitude de mesure sur ledit ratio molaire étant de 0,2%. Dans une variante avantageuse, le ratio molaire $K_2O$ / ($K_2O$ + BaO + $Na_2O$) est égal à 8,3%. Dans la présente invention, le ratio molaire $K_2O$ / ($K_2O$ + BaO + $Na_2O$) est exprimé en pourcent du nombre de moles de $K_2O$ par rapport à la somme du nombre de moles de ($K_2O$ + BaO + $Na_2O$).

La teneur en oxyde de sodium $Na_2O$ est de manière préférée inférieure à 0,2% en poids par rapport la masse totale de l'adsorbant.

La teneur totale en oxydes d'ions alcalins ou alcalino-terreux autres que l'oxyde de baryum BaO, l'oxyde de potassium $K_2O$ et l'oxyde de sodium $Na_2O$ est avantageusement inférieure à 1% en poids, de préférence entre 0 et 0,5% en poids, et de manière très préférée entre 0 et 0,3% en poids, par rapport à la masse totale de l'adsorbant.

Les cristaux de zéolithes X ont avantageusement un rapport atomique Si/Al compris entre 1,00 et 1,50, de préférence entre 1,05 et 1,50 et de manière encore préférée entre 1,10 et 1,50.

Le diamètre moyen en nombre des cristaux de zéolithes X est inférieur ou égal à 1,5 $\mu$m, de préférence compris entre 0,1 $\mu$m et 1,2 $\mu$m, de manière plus préférée compris entre 0,1 $\mu$m et 1,0 $\mu$m.

La perte au feu de l'adsorbant selon l'invention, mesurée à 950 °C selon la norme NF EN 196-2 est avantageusement comprise entre 4,0 et 7,7% et de préférence entre 4,5 et 6,5 % et de manière très préférée entre 4,8 et 6% en poids.

Le diamètre moyen en nombre de l'adsorbant selon l'invention peut être compris entre 0,2 mm et 2 mm, en particulier entre 0,2 mm et 0,8 mm et de préférence entre 0,2 mm et 0,65 mm.

L'invention concerne également un procédé de préparation d'un adsorbant tel que décrit ci-dessus, comprenant au moins les étapes de :

a) agglomération d'une poudre de cristaux de la zéolithe X avec un liant, et mise en forme, puis séchage et calcination,
b) zéolithisation éventuelle du liant,
c) échange cationique de l'aggloméré par mise en contact avec une solution d'ions baryum, ou d'ions potassium, ou d'ions baryum et d'ions potassium, à des concentrations comprises entre 0,05 M et 1,5 M, de préférence comprises entre 0,1 M et 1,2 M,
d) échange cationique au potassium lorsque l'étape c) d'échange est réalisée avec une solution d'ions baryum seuls, ou échange cationique au baryum lorsque l'étape c) d'échange est réalisée avec une solution d'ions potassium seuls,
e) puis lavage et séchage du produit ainsi traité,
f) activation de l'adsorbant zéolithique ainsi obtenu.

De préférence, le procédé de préparation de l'adsorbant met en oeuvre une étape b) de zéolithisation du liant.

L'invention concerne également l'utilisation dudit adsorbant selon l'invention dans les procédés de :

- séparation de coupes d'isomères aromatiques en C8 et notamment des xylènes,
- séparation d'isomères de toluène substitué tels que nitrotoluène, diéthyltoluène, toluènediamine,
- séparation des crésols,
- séparation des alcools polyhydriques,

et notamment pour la séparation de *para*-xylène à partir de coupes d'isomères aromatiques à 8 atomes de carbone.

L'invention concerne également un procédé de récupération de *para*-xylène à partir de coupes d'isomères d'hydrocarbures aromatiques contenant 8 atomes de carbone, en phase liquide, par adsorption du *para*-xylène au moyen dudit adsorbant selon l'invention en présence d'un désorbant, de préférence choisi parmi le toluène et le *para*-diéthylbenzène.

Ledit procédé peut être de type lit mobile simulé, de préférence à contre-courant simulé.

L'invention concerne également un procédé de récupération de *para*-xylène à partir de coupes d'isomères d'hydrocarbures aromatiques contenant 8 atomes de carbone, en phase gazeuse, par adsorption du *para*-xylène au moyen dudit adsorbant selon l'invention en présence d'un désorbant, de préférence choisi parmi le toluène et le *para*-diéthylbenzène.

Dans ce qui va suivre, et à moins d'une autre indication, les bornes d'un domaine de valeurs sont comprises dans ce domaine, notamment dans les expressions « compris entre » et « allant de ... à ... ».

DESCRIPTION DETAILLEE DE L'INVENTION

La présente invention a ainsi pour premier objet des adsorbants zéolithiques à base de zéolithe X. Ces adsorbants sont particulièrement adaptés pour une utilisation dans un procédé de séparation du *para*-xylène en phase liquide, de préférence de type contre-courant simulé.

Ainsi, la présente invention concerne un adsorbant zéolithique comprenant des cristaux de zéolithe X et comprenant du baryum, du potassium et du sodium, dans lequel la teneur en oxyde de sodium $Na_2O$ est inférieure à 0,3% en poids par rapport à la masse totale de l'adsorbant et dans lequel le ratio molaire $K_2O$ / ($K_2O$ + BaO + $Na_2O$) est compris entre 8,0% et 8,6%, de préférence entre 8,2% et 8,4% et de manière plus préférée est égal à 8,3%, l'incertitude de mesure sur ledit ratio molaire étant de 0,2%.

Les adsorbants selon l'invention peuvent également comprendre une phase non zéolithique, c'est-à-dire une phase non cristalline qui est essentiellement inerte vis-à-vis de l'adsorption. Dans le cas où l'adsorbant selon l'invention comprend une phase non zéolithique, le ratio molaire $K_2O$ / ($K_2O$ + BaO + $Na_2O$) prend en compte les oxydes compris dans ladite phase non zéolithique.

La teneur en oxyde de sodium $Na_2O$ dans l'adsorbant selon l'invention est de manière préférée inférieure à 0,2% en poids par rapport la masse totale de l'adsorbant.

La teneur totale en oxydes d'ions alcalins ou alcalino-terreux autres que l'oxyde de baryum BaO, l'oxyde de potassium $K_2O$ et l'oxyde de sodium $Na_2O$ dans l'adsorbant selon l'invention est avantageusement inférieure à 1% en poids, de préférence entre 0 et 0,5% en poids, et de manière très préférée entre 0 et 0,3% en poids, par rapport à la masse totale de l'adsorbant.

L'adsorbant zéolithique selon la présente invention est un adsorbant à base de cristaux de zéolithe FAU de type X. Par « zéolithe X », on entend les zéolithes dont le ratio atomique Si/Al est compris entre 1,00 et 1,50, de préférence entre 1,05 et 1,50 et de manière encore plus préférée entre 1,10 et 1,50.

Parmi les zéolithes X, il est maintenant communément admis de reconnaître deux sous-groupes dénommés zéolithes LSX et zéolithes MSX. Les zéolithes LSX présentent un ratio atomique Si/Al égal à environ 1 et les zéolithes MSX présentent un ratio atomique Si/Al compris entre environ 1,05 et environ 1,15.

Dans l'adsorbant zéolithique de la présente invention, et selon un mode de réalisation préféré, par « zéolithe FAU de type X », on entend les zéolithes FAU de type X définies ci-dessus, ces dites zéolithes étant à porosité hiérarchisée c'est-à-dire, les zéolithes de type X à porosité hiérarchisée (ou zéolithe XPH), les zéolithes de type MSX à porosité hiérarchisée (ou MSXPH) et les zéolithes de type LSX à porosité hiérarchisée (ou LSXPH), et plus particulièrement les zéolithes FAU à porosité hiérarchisée et de rapport atomique Si/Al compris entre 1,00 et 1,50, de préférence entre 1,05 et 1,50, de préférence encore entre 1,05 et 1,40, et de manière encore plus préférée, entre 1,15 et 1,40.

L'invention comprend également les adsorbants zéolithiques comprenant des mélanges de deux ou plusieurs zéolithes FAU à porosité hiérarchisée telles qu'elles viennent d'être définies.

Par « zéolithe à porosité hiérarchisée », on entend une zéolithe possédant à la fois des micropores et des mésopores, autrement dit une zéolithe à la fois microporeuse et mésoporeuse. Par « zéolithe mésoporeuse », on entend une zéolithe dont les cristaux zéolithiques microporeux présentent, conjointement à la microporosité, des cavités internes de taille nanométrique (mésoporosité), facilement identifiables par observation au moyen d'un Microscope Électronique à Transmission (MET ou « TEM » en langue anglaise), comme décrit par exemple dans US 7 785 563 : l'observation par microscopie électronique à transmission (MET) permet de vérifier si les cristaux zéolithiques sont des cristaux de zéolithe pleins (i.e. non mésoporeux) ou des agrégats de cristaux de zéolithes pleins ou des cristaux mésoporeux ou des agrégats de cristaux mésoporeux.

La structure cristalline de la zéolithe FAU de type X dans l'adsorbant zéolithique de la présente invention, est identifiable par diffraction des rayons X (connue de l'homme du métier sous l'acronyme DRX).

Selon un mode de réalisation préféré, l'adsorbant zéolithique présente un rapport atomique Si/Al compris entre 1,00 et 2,00 de préférence entre 1,00 et 1,80, de préférence encore entre 1,15 et 1,80, et de manière encore plus préférée entre 1,15 et 1,60.

Dans le présent document, on emploie l'appellation « diamètre moyen en nombre » ou bien « taille » pour les cristaux de zéolithe et pour les agglomérés zéolithiques. La méthode de mesure de ces grandeurs est explicitée plus loin dans la description. Selon un mode de réalisation préféré de la présente invention, le diamètre moyen en nombre des cristaux de zéolithes X est inférieur ou égal à 1,5 $\mu$m, de préférence compris entre 0,1 $\mu$m et 1,2 $\mu$m, de manière plus préférée compris entre 0,1 $\mu$m et 1,0 $\mu$m.

L'adsorbant zéolithique de l'invention est de préférence sous la forme d'un aggloméré, c'est-à-dire qu'il est constitué de cristaux de zéolithe(s) et d'au moins une phase non zéolithique qui peut comprendre un liant d'agglomération permettant la cohésion des cristaux entre eux. Ainsi l'adsorbant zéolithique de l'invention est souvent dénommé « aggloméré » dans le présent exposé.

La fraction massique de zéolithe X dans l'adsorbant selon la présente invention peut être d'au moins 80% en poids par rapport au poids total de l'adsorbant, de préférence d'au moins 90%, cette fraction massique pouvant aller jusqu'à 100% et typiquement jusqu'à 99,5% en poids.

Selon un mode de réalisation préféré, l'adsorbant zéolithique selon l'invention présente une perte au feu mesurée à 950 °C selon la norme NF EN 196-2 comprise entre 4,0% et 7,7%, préférence entre 4,5 et 6,5 % et avantageusement entre 4,8 et 6%.

L'adsorbant zéolithique selon la présente invention présente préférentiellement une résistance mécanique généralement supérieure ou égale à 1,8 MPa, typiquement supérieure ou égale à 2,1 MPa. Cette résistance mécanique est mesurée par la méthode Shell série SMS1471-74 adaptée pour des agglomérés de taille inférieure à 1,6 mm.

La capacité d'adsorption est quant à elle mesurée par mesure du volume microporeux de l'adsorbant évalué d'après l'équation de Dubinin-Raduskevitch par adsorption d'azote ($N_2$) à une température de 77K, après dégazage sous vide à 300°C pendant 16 heures. Le volume microporeux des adsorbants zéolithiques de l'invention a ainsi été mesuré comme étant supérieur à 0,250 cm³/g, typiquement dans une plage allant de 0,256 cm³/g à 0,288 cm³/g.

Selon un autre aspect, l'invention concerne un procédé de préparation des agglomérés zéolithiques tels qu'ils viennent d'être définis, procédé qui comprend au moins les étapes de :

a) agglomération de cristaux (poudre) de zéolithe X avec un liant, et mise en forme d'aggloméré, puis séchage et calcination,

b) zéolithisation éventuelle dudit liant, de préférence par action d'une solution basique alcaline,

c) échange cationique de l'aggloméré par mise en contact avec une solution d'ions baryum, ou d'ions potassium, ou d'ions baryum et d'ions potassium, à des concentrations comprises entre 0,05 M et 1,5 M, de préférence comprises entre 0,1 M et 1,2 M,

d) échange cationique au potassium lorsque l'étape c) d'échange est réalisée avec une solution d'ions baryum seuls, ou échange cationique au baryum lorsque l'étape c) d'échange est réalisée avec une solution d'ions potassium seuls,

e) puis lavage et séchage du produit ainsi traité,

f) activation de l'aggloméré zéolithique ainsi obtenu.

De préférence, le procédé de préparation des agglomérés zéolithiques met en oeuvre une étape b) de zéolithisation du liant.

La taille des cristaux de zéolithe X utilisés à l'étape a) est mesurée par observation au microscope électronique à balayage (MEB) ou par observation au microscope électronique en transmission (MET). Cette observation MEB ou MET permet également de confirmer la présence de phase non zéolithique comprenant par exemple le liant ou le liant résiduel non converti lors de l'étape optionnelle de zéolithisation ou toute autre phase amorphe dans les agglomérés.

Selon un mode de réalisation, la zéolithe X utilisée à l'étape a) comprend, de préférence est, une zéolithe FAU de type X à porosité hiérarchisée. Les cristaux de zéolithe FAU de type X à porosité hiérarchisée présentant une importante surface externe peuvent être obtenus selon diverses méthodes connues de l'homme du métier et par exemple selon la synthèse décrite par Inayat et coll. (Angew. Chem. Int. Ed., (2012), 51, 1962-1965).

Il est également possible de préparer lesdits cristaux par synthèse par ensemencement et/ou par ajustement des conditions opératoires de synthèse tels que le rapport $SiO_2/Al_2O_3$, la teneur en sodium et l'alcalinité du mélange de synthèse ou encore selon des procédés de post-traitement de cristaux de zéolithe FAU de type X conventionnels et connus de l'homme du métier.

Les procédés de post-traitements consistent généralement à éliminer des atomes du réseau zéolithique déjà formé, soit par un ou plusieurs traitements acides qui désaluminent le solide, traitement(s) suivi(s) par un ou plusieurs lavage(s) à la soude (NaOH) afin d'éliminer les résidus aluminiques formés, comme décrit par exemple par D. Verboekend et coll. (Adv. Funct. Mater., 22, (2012), pp. 916-928), soit encore par des traitements qui associent l'action d'un acide et celle d'un agent structurant qui améliorent l'efficacité du traitement acide, comme décrit par exemple dans la demande WO2013/106816.

L'agglomération et la mise en forme (étape a) peuvent être réalisées selon toutes les techniques connues de l'homme de l'art, telles qu'extrusion, compactage, agglomération, et autres. Les proportions de liant d'agglomération, éventuellement zéolithisable, (voir définition plus loin) et de zéolithe(s) mises en oeuvre sont typiquement celles de l'art antérieur, c'est-à-dire de 5 parties à 20 parties en poids de liant pour 95 parties à 80 parties en poids de zéolithe. Les agglomérés issus de l'étape a), qu'ils soient sous forme de billes, d'extrudés ou autres, ont en général un diamètre moyen en nombre (ou leur plus grande dimension lorsqu'ils ne sont pas sphériques) compris entre 0,2 mm et 2 mm, et en particulier comprise entre 0,2 mm et 0,8 mm et de préférence entre 0,2 mm et 0,65 mm.

À l'issue de l'étape a), les particules d'agglomérés les plus fines peuvent être éliminées par cyclonage et/ou tamisage et/ou les particules trop grosses par tamisage ou concassage, dans le cas d'extrudés, par exemple.

Le liant d'agglomération mis en oeuvre à l'étape a) peut être zéolithisable. Il contient alors au moins 80 % de préférence, au moins 90%, de préférence encore au moins 95%, plus particulièrement au moins 96%, en poids, d'argile zéolithisable et peut également contenir d'autres liants minéraux tels que bentonite, attapulgite, et autres. Par argile zéolithisable, on entend une argile ou un mélange d'argiles qui sont susceptibles d'être converties en matière zéolithique (c'est-à-dire matière active au sens de l'adsorption), le plus souvent par action d'une solution basique alcaline. L'argile zéolithisable appartient en général à la famille des kaolins, kaolinites, nacrites, dickites, halloysite et/ou métakaolins. Le kaolin est préféré et le plus couramment utilisé.

D'autres argiles telles que notamment la sépiolite ou l'attapulgite peuvent également être utilisées.

Dans tous les cas, les argiles peuvent être utilisées dans leur état brut ou peuvent être préalablement soumises à un ou plusieurs traitements, par exemple choisis parmi calcination, traitement à l'acide, modification chimique, et autres.

La poudre de zéolithe X mise en oeuvre à l'étape a) peut être issue de la synthèse de cristaux de zéolithe X comprenant majoritairement, voir exclusivement des cations sodium, par exemple les zéolithes NaX, mais on ne sortirait pas du cadre de l'invention en utilisant une poudre ayant subi un ou plusieurs échanges cationiques, après sa synthèse et avant sa mise en œuvre à l'étape a).

Lors de l'étape a), outre la poudre de zéolithe X et le liant, un ou plusieurs additifs peuvent également être ajoutés, par exemple des additifs destinés à faciliter l'agglomération ou à améliorer le durcissement des agglomérés formés tels que la lignine, l'amidon, la carboxyméthylcellulose, et autres additifs connus de l'homme du métier. De la silice peut également être ajoutée. La source éventuelle de silice peut être de tout type connu de l'homme du métier, spécialiste de la synthèse de zéolithes, par exemple de la silice colloïdale, des diatomées, de la perlite, des cendres de calcination (« fly ash » en langue anglaise), du sable, ou toute autre forme de silice solide.

Après le séchage à l'étape a), la calcination est menée à une température en général comprise entre 500°C et 600°C. Dans le cas où la mise en forme est réalisée avec une argile zéolithisable, cette étape permet de transformer l'argile zéolithisable, typiquement le kaolin, en métakaolin qui peut après être converti en zéolithe lors de l'étape de zéolithisation (étape b)). Le principe en est exposé dans « Zeolite Molecular Sieves » de D.W. Breck, John Wiley and Sons, New York, (1973), p. 314-315.

La zéolithisation du liant d'agglomération est pratiquée selon toute méthode connue de l'homme du métier et peut par exemple être réalisée par immersion du produit de l'étape a) dans une solution basique alcaline, en général aqueuse, par exemple une solution aqueuse d'hydroxyde de sodium et/ou d'hydroxyde de potassium.

En règle générale, la concentration de la solution alcaline de zéolithisation est de préférence comprise entre 0,5 M et 5 M. La zéolithisation s'opère de préférence à chaud, à une température supérieure à la température ambiante, et typiquement à des températures de l'ordre de 80°C à 100°C, par exemple comprises entre la température ambiante (soit environ 20°C) et la température d'ébullition de la solution alcaline de zéolithisation. La durée du processus de zéolithisation est généralement comprise entre quelques dizaines de minutes et quelques heures, de préférence entre environ 1 heure et 8 heures.

Les étapes c) et d) d'échange au baryum et/ou au potassium des cations de la zéolithe X s'effectuent selon les méthodes classiques connues de l'homme du métier, et le plus souvent par mise en contact des agglomérés issus de l'étape a) ou de l'étape b) avec un sel, tel que le chlorure de baryum ($BaCl_2$) pour l'échange au baryum et/ou le chlorure de potassium (KCl) pour l'échange au potassium, en solution aqueuse à une température comprise entre la température ambiante et 100°C, et de préférence comprise entre 80°C et 100°C. Pour obtenir rapidement des teneurs en oxyde de sodium faibles, i.e. inférieures à 1%, on préfère opérer avec un large excès d'ions baryum et/ou potassium par rapport aux cations de la zéolithe que l'on souhaite échanger, typiquement un excès de l'ordre de 10 à 12, avantageusement en procédant par échanges successifs. Afin d'atteindre le ratio molaire $K_2O$ / ($K_2O$ + BaO + $Na_2O$) comprise entre 8,0 et 8,6%, un ou plusieurs échange(s) ionique(s) sont réalisés en utilisant des solutions aqueuses d'ions baryum et des solutions aqueuses d'ions potassium, par exemple chlorure de potassium et chlorure de baryum, à des concentrations comprises entre 0,05 M et 1,5 M, de préférence comprises entre 0,1 M et 1,2 M.

Selon un mode de réalisation particulier, au moins un échange ionique est réalisé en utilisant une solution aqueuse d'ions baryum et d'ions potassium (correspondant à l'étape c)). Dans ce mode de réalisation, l'étape d) du procédé de l'invention n'est pas réalisée. Ce mode de réalisation est le mode préféré.

Selon un autre mode de réalisation de l'invention, au moins un échange ionique est tout d'abord réalisé en utilisant une solution aqueuse d'ions baryum (correspondant à l'étape c)), puis au moins un échange ionique est réalisé en utilisant une solution aqueuse d'ions potassium (correspondant à l'étape d)).

Selon un autre mode de réalisation de l'invention, au moins un échange ionique est tout d'abord réalisé en utilisant une solution aqueuse d'ions potassium (correspondant à l'étape c)), puis au moins un échange ionique est réalisé en utilisant une solution aqueuse d'ions baryum (correspondant à l'étape d)).

Chaque étape d'échange peut être réalisée une ou plusieurs fois.

Le ou les échanges sont réalisés selon les techniques bien connues de l'homme du métier, par exemple à des températures comprises entre la température ambiante (soit environ 20°C) et 100°C, de préférence entre 80 et 100°C, généralement à pression atmosphérique, le ou les échanges étant généralement effectués pendant des périodes allant de quelques minutes à quelques heures, de préférence typiquement entre 30 minutes et 3 heures.

L'ajustement du ratio molaire $K_2O$ / ($K_2O$ + BaO + $Na_2O$) est réalisé selon toutes méthodes connues de l'homme du métier, et par exemple en réalisant un échange avec un large excès d'ions baryum afin d'obtenir rapidement des teneurs faibles en oxyde de sodium $Na_2O$, i.e. inférieures à 0,3%, et en réalisant ensuite un échange à l'aide d'une solution aqueuse d'ions potassium contenant la quantité molaire nécessaire d'ions potassium afin d'obtenir le ratio molaire $K_2O$ / ($K_2O$ + BaO + $Na_2O$) souhaité.

Comme indiqué précédemment, il est également possible d'agglomérer à l'étape a) de la poudre de zéolithe X contenant déjà des ions potassium (pré-échange des cations présents dans la zéolithe X de départ, typiquement cations sodium, par des ions potassium avant l'étape a)) et s'affranchir ou non des échanges au potassium lors des étapes c) et/ou d).

On procède ensuite à un lavage, généralement et de préférence à l'eau, puis d'un séchage de l'aggloméré ainsi obtenu.

L'activation qui suit le séchage, est conduite de manière classique, selon les méthodes connues de l'homme du métier, par exemple à une température en général comprise entre 100°C et 400°C, de préférence entre 200°C et 300°C. Cette étape f) d'activation a pour but de fixer la teneur en eau, ainsi que la perte au feu de l'adsorbant de façon optimale pour l'utilisation envisagée. On procède en général par activation thermique qu'on exécute préférentiellement entre 200°C et 300°C pendant une durée déterminée en fonction de la teneur en eau et de la perte au feu souhaitées, typiquement de 1 à 6 heures.

La présente invention concerne également les utilisations des adsorbants zéolithiques décrits ci-dessus comme agents d'adsorption susceptibles de remplacer avantageusement les agents d'adsorption décrits dans la littérature pour les utilisations listées ci-dessous :

- séparation de coupes d'isomères aromatiques en C8 et notamment des xylènes,
- séparation d'isomères de toluène substitué tels que nitrotoluène, diéthyltoluène, toluènediamine,
- séparation des crésols,
- la séparation des alcools polyhydriques, tels que les sucres.

L'invention concerne notamment un procédé de récupération de para-xylène à haute pureté à partir de coupes d'isomères aromatiques à 8 atomes de carbone consistant à utiliser, comme agent d'adsorption du *para*-xylène, un adsorbant zéolithique selon l'invention, mis en oeuvre dans des procédés en phase liquide mais aussi en phase gazeuse. Par *para*-xylène de haute pureté, nous entendons un produit approprié pour une utilisation dans la production d'acide téréphtalique ou de téréphtalate de diméthyle, c'est à dire une pureté d'au moins 99,5% en poids, de préférence au moins 99,7% en poids, de préférence au moins 99,8% en poids et plus préférablement encore au moins 99,9% en poids. La pureté du *para*-xylène peut-être déterminée par des méthodes chromatographiques. Une méthode de chromatographie en phase gazeuse utilisable à la fois pour la détermination de la pureté du *para*-xylène et des quantités spécifiques d'impuretés est la méthode ASTM D-3798.

On peut ainsi séparer le produit désiré (*para*-xylène) par chromatographie liquide d'adsorption préparative (en batch), et avantageusement en continu en lit mobile simulé, c'est-à-dire à contre-courant simulé ou à co-courant simulé, et plus particulièrement à contre-courant simulé.

Le procédé de récupération de *para*-xylène selon l'invention utilisant l'adsorbant décrit selon l'invention présente l'avantage de maximiser la productivité, c'est-à-dire de maximiser le débit de charge à traiter. Ceci est particulièrement vrai dans les conditions opératoires d'unité industrielle d'adsorption de type contre-courant simulé suivantes :

- nombre de lits : 6 à 30,
- nombre de zones : au moins 4 zones de fonctionnement, chacune étant localisées entre un point d'alimentation et un point de soutirage,
- température comprise entre 100°C et 250°C, de préférence entre 150°C et 190°C,
- pression de l'unité industrielle comprise entre la pression de bulle des xylènes à la température du procédé et 3 MPa,
- rapport des débits désorbant/charge compris entre 0,7 et 2,5, par exemple entre 0,9 et 1,8 pour une unité d'adsorption seule (stand alone) et entre 0,7 et 1,4 pour une unité d'adsorption combinée à une unité de cristallisation,
- taux de recyclage (i.e. ratio du débit de recyclage moyen (moyenne des débits de zones pondérée du nombre de lits par zones) sur le débit de charge) compris entre 2,5 et 12, de préférence entre 3,5 et 6.

On pourra sur ce sujet se référer à l'enseignement des brevets US 2 985 589, US 5 284 992 et US 5 629 467.

Les conditions opératoires d'une unité industrielle d'adsorption à co-courant simulé sont en général les mêmes que celles fonctionnant à contre-courant simulé, à l'exception du taux de recyclage qui est en général compris entre 0,8 et 7. On pourra sur cet aspect se référer aux brevets US 4 402 832 et US 4 498 991.

Le solvant de désorption peut être tout désorbant connu de l'homme du métier et dont le point d'ébullition est inférieur à celui de la charge, tel que le toluène mais aussi un désorbant dont le point d'ébullition est supérieur à celui de la charge, tel que le *para*-diéthylbenzène (PDEB). La sélectivité des adsorbants selon l'invention pour l'adsorption du para-xylène contenu dans des coupes aromatiques en C8 est optimale lorsque leur perte au feu mesurée à 950°C est comprise en général entre 4,0% et 7,7%, et de préférence entre 4,5% et 6,5%, et de manière très préférée entre 4,8% et 6,0%.

TECHNIQUES DE CARACTERISATION

**Granulométrie des cristaux** :

L'estimation du diamètre moyen en nombre des cristaux de zéolithe X utilisées à l'étape a) et des cristaux de zéolithe X contenue dans les agglomérés est réalisée par observation au microscope électronique à balayage (MEB) ou par observation au microscope électronique en transmission (MET).

Afin d'estimer la taille des cristaux de zéolithe sur les échantillons, on effectue un ensemble de clichés à un grossissement d'au moins 5000. On mesure ensuite le diamètre d'au moins 200 cristaux à l'aide d'un logiciel dédié, par exemple le logiciel Smile View de l'éditeur LoGraMi. La précision est de l'ordre de 3%.

**Analyse chimique des adsorbants zéolithiques - rapport Si/Al et $K_2O$ / ($K_2O$ + BaO + $Na_2O$)**:

Une analyse chimique élémentaire du produit final obtenu à l'issue des étapes a) à f) décrites précédemment, peut être réalisée selon différentes techniques analytiques connues de l'homme du métier. Parmi ces techniques, on peut citer la technique d'analyse chimique par fluorescence de rayons X telle que décrite dans la norme NF EN ISO 12677 : 2011 sur un spectromètre dispersif en longueur d'onde (WDXRF), par exemple Tiger S8 de la société Bruker.

La fluorescence X est une technique spectrale non destructive exploitant la photoluminescence des atomes dans le domaine des rayons X, pour établir la composition élémentaire d'un échantillon. L'excitation des atomes généralement par un faisceau de rayons X ou par bombardement avec des électrons, génère des radiations spécifiques après retour à l'état fondamental de l'atome. Le spectre de fluorescence X a l'avantage de dépendre très peu de la combinaison chimique de l'élément, ce qui offre une détermination précise, à la fois quantitative et qualitative. On obtient de manière classique après étalonnage pour chaque oxyde une incertitude de mesure inférieure à 0,4% en poids. Dans la présente invention les teneurs en baryum, en silicium et en aluminium sont de préférence mesurées par la méthode fluorescence X décrite ci-dessus.

En revanche pour les éléments plus légers en termes poids atomique tels que le sodium ou le potassium présents dans l'adsorbant, on préférera pour plus de précision la spectrométrie d'émission atomique avec plasma induit par haute fréquence (ICP-OES pour Inductively Coupled Plasma-Optical Emission Spectroscopy selon la terminologie anglo-saxonne) selon la norme UOP 961-12.

L'ICP est une méthode d'analyse par spectrométrie d'émission atomique dont la source est un plasma généré par couplage inductif. Cette méthode est également couramment employée pour déterminer les teneurs en divers éléments tels que le silicium, l'aluminium, le potassium le sodium et le baryum. Dans la présente invention les teneurs en sodium et en potassium sont de préférence mesurées par la méthode ICP selon la norme UOP 961-12. On obtient dans ce cas pour le sodium une incertitude sur la mesure inférieure à 0,01% pour la teneur en poids de l'oxyde de sodium dans l'adsorbant et pour le potassium une incertitude sur la mesure inférieure à 0,02% pour la teneur en poids de l'oxyde de potassium dans l'adsorbant.

Ces analyses chimiques élémentaires permettent à la fois de vérifier le rapport atomique Si/Al de la zéolithe au sein de l'aggloméré, et de vérifier la qualité de l'échange ionique décrit à l'étape c) et à l'étape d). Dans la description de la présente invention, l'incertitude de mesure du ratio atomique Si/Al est de 0,05.

La qualité de l'échange ionique est liée au nombre de mole d'oxyde de sodium, $Na_2O$, restant dans l'aggloméré zéolithique après échange. Plus précisément, le taux d'échange par les ions baryum est déterminé par le ratio entre le nombre de moles d'oxyde de baryum, BaO, et le nombre de moles de l'ensemble (BaO + $K_2O$ + $Na_2O$). De même, le taux d'échange par les ions potassium est déterminé par le ratio entre le nombre de moles d'oxyde de potassium, $K_2O$, et le nombre de moles de l'ensemble (BaO + $K_2O$ + $Na_2O$). BaO, $K_2O$ et $Na_2O$ sont exprimés sous forme d'oxydes. Le taux d'échange total par les ions baryum et potassium est estimé à partir de la somme des deux taux d'échange précédemment décrits, correspondant au rapport entre la somme du nombre de moles d'oxyde de baryum et oxyde de potassium (BaO + $K_2O$) et le nombre de moles de l'ensemble (BaO + $K_2O$ + $Na_2O$). Il est à noter que les teneurs en différents oxydes sont donnés, en pourcentage en poids par rapport au poids total de l'adsorbant zéolithique anhydre. Dans la description de la présente invention, l'incertitude de mesure sur le ratio molaire $K_2O$ / ($K_2O$ + BaO + $Na_2O$) est de 0,2%.

**Granulométrie des adsorbants zéolithiques** :

La détermination du diamètre moyen en nombre des adsorbants zéolithiques obtenus à l'issus de l'étape a) d'agglomération et de mise en forme est effectuée par analyse de la distribution granulométrique d'un échantillon d'aggloméré par imagerie selon la norme ISO 13322-2:2006, en utilisant un tapis roulant permettant à l'échantillon de passer devant l'objectif de la caméra.

Le diamètre moyen en nombre est ensuite calculé à partir de la distribution granulométrique en appliquant la norme ISO 9276-2:2001. Dans le présent document, on emploie l'appellation « diamètre moyen en nombre » ou bien « taille » pour les agglomérés zéolithiques. La précision est de l'ordre de 0,01 mm pour la gamme de taille d'agglomérés de l'invention.

**Résistance mécanique des adsorbants zéolithiques** :

La technique de caractérisation de la résistance mécanique représentative de l'écrasement de l'adsorbant au sein d'un lit ou d'un réacteur est la technique de caractérisation de la résistance mécanique en lit, telle que décrite dans la méthode Shell série SMS1471-74 (Shell Method Series SMS1471-74 Détermination of Bulk Crushing Strength of Catalysts. Compression-Sieve Method") , associée à l'appareil "BCS Tester" commercialisé par la société Vinci Technologies. Cette méthode, initialement destinée à la caractérisation de catalyseurs de 3 à 6 mm est basée sur l'utilisation d'un tamis de 425 $\mu$m qui va permettre notamment de séparer les fines créées lors de l'écrasement. L'utilisation d'un tamis de 425 $\mu$m reste adaptée pour des particules de diamètre supérieur à 1,6mm, mais doit être adaptée selon la granulométrie des adsorbants zéolithiques que l'on cherche à caractériser. La norme ASTM D7084-04 qui décrit également une méthode de mesure de la résistance à l'écrasement en lit de catalyseurs ("Détermination of Bulk Crush Strength of Catalysts and Catalyst Carriers") définit le passage du tamis à utiliser comme étant égal à la moitié du diamètre des particules de catalyseurs à caractériser. La méthode prévoit une étape préliminaire de tamisage de l'échantillon de catalyseurs ou adsorbants à caractériser. Si une quantité égale à 10% en poids de l'échantillon passe à travers la grille, un tamis de passage plus petit sera utilisé.

Les agglomérés de la présente invention, généralement sous forme de billes ou d'extrudés, ont en général un diamètre moyen en nombre ou une longueur, i.e. plus grande dimension dans le cas des agglomérés non sphériques, comprise entre 0,2 mm et 2 mm, et en particulier comprise entre 0,2 mm et 0,8 mm et de préférence entre 0,2 mm et 0,65 mm. Par conséquent, un tamis adapté tel que moins de 10% en poids de l'échantillon passe à travers la grille lors d'une étape préalable de tamisage est utilisé à la place du tamis de 425 $\mu$m mentionné dans la méthode Shell standard SMS1471-74.

Le protocole de mesure est le suivant : un échantillon de 20 cm$^3$ d'adsorbants agglomérés, préalablement tamisé avec le tamis adapté et préalablement séché à l'étuve pendant au moins 2 heures à 250°C (au lieu de 300°C mentionné dans la méthode Shell standard SMS1471-74), est placé dans un cylindre métallique de section interne connue. Une force croissante est imposée par paliers sur cet échantillon par l'intermédiaire d'un piston, à travers un lit de 5 cm$^3$ de billes d'acier afin de mieux répartir la force exercée par le piston sur les agglomérés d'adsorbants (utilisation de billes de 2 mm de diamètre pour des particules de forme sphérique de diamètre strictement inférieur à 1,6 mm). Les fines obtenues aux différents paliers de pression sont séparées par tamisage (avec tamis adapté) et pesées.

La résistance à l'écrasement en lit est déterminée par la pression en mégaPascal (MPa) pour laquelle la quantité de fines cumulées passant à travers le tamis s'élève à 0,5% pondéral de l'échantillon. Cette valeur est obtenue en traçant sur un graphique la masse de fines obtenue en fonction de la force appliquée sur le lit d'adsorbant et en interpolant à 0,5 % massique de fines cumulées. La résistance mécanique à l'écrasement en lit est typiquement comprise entre quelques centaines de kPa et quelques dizaines de MPa et généralement comprise entre 0,3 MPa et 4 MPa. La précision est de manière classique inférieure à 0,1 MPa.

**Détermination des fractions zéolithiques des adsorbants zéolithiques** :

La nature et la quantité des différentes fractions zéolithiques sont déterminées par analyse par diffraction de rayons X, connue de l'homme du métier sous l'acronyme DRX. Cette analyse est réalisée sur un appareil de la marque Bruker, puis la quantité des fractions zéolithiques est évaluée au moyen du logiciel TOPAS de la société Bruker.

**Volume microporeux** :

La cristallinité des agglomérés est également évaluée par mesure de leur volume microporeux en le comparant à celui d'une référence appropriée (zéolithe 100% cristalline dans des conditions de traitements cationiques identiques ou zéolithe théorique). Ce volume microporeux est déterminé à partir de la mesure de l'isotherme d'adsorption de gaz, tel que l'azote, à sa température de liquéfaction. Préalablement à l'adsorption, l'adsorbant zéolithique est dégazé entre 300°C - 450°C pendant une durée de 9 heures à 16 heures, sous vide (P < 6,7.10$^{-4}$ Pa). La mesure de l'isotherme d'adsorption d'azote à 77K est ensuite effectuée sur un appareil de type ASAP 2010 M de Micromeritics, en prenant au moins 35 points de mesure à des pressions relatives de rapport $P/P_0$ compris entre 0,002 et 1. Le volume microporeux est déterminé selon Dubinin et Raduskevitch à partir de l'isotherme obtenue, en appliquant la norme ISO 15901-3:2007. Le volume microporeux évalué selon Dubinin et Raduskevitch s'exprime en cm$^3$ d'adsorbat liquide par gramme d'adsorbant. L'incertitude de mesure est de $\pm$ 0,003.

**Perte au feu des adsorbants zéolithiques** :

La perte au feu est déterminée en atmosphère oxydante, par calcination de l'échantillon à l'air à une température de 950°C ± 25°C, comme décrit dans la norme NF EN 196-2 (avril 2006). L'écart type de mesure est inférieur à 0,1%.

**Caractérisation de l'adsorption en phase liquide par perçage** :

La technique utilisée pour caractériser l'adsorption de molécules en phase liquide sur un solide poreux est la technique dite de perçage, décrite par Ruthven dans « Principles of Adsorption and Adsorption Processes » (Chapitres 8 et 9, John Wiley & Sons, 1984) qui définit la technique des courbes de perçage (« breakthrough curves ») comme l'étude de la réponse à l'injection d'un échelon de constituants adsorbables. L'analyse du temps moyen de sortie (premier moment) des courbes de perçage fournit une information sur les quantités adsorbées et permet également d'évaluer les sélectivités, c'est-à-dire le facteur de séparation, entre deux constituants adsorbables. L'injection d'un constituant non adsorbable utilisé comme traceur est conseillée pour l'estimation des volumes non-sélectifs. L'analyse de la dispersion (second moment) des courbes de perçage, permet d'évaluer la hauteur équivalente de plateaux théoriques, basée sur la représentation d'une colonne par un nombre fini de réacteurs hypothétiques idéalement agités (étages théoriques), qui est une mesure directe de la dispersion axiale et de la résistance au transfert de matière du système.

EXEMPLES

**Préparation des adsorbants zéolithiques**

On prépare un mélange homogène et on agglomère 800 g de cristaux de zéolithe NaX selon le mode opératoire décrit dans la demande de brevet FR2 999 098 (synthèse de l'exemple B) avec 105 g de kaolin (exprimés en équivalent calciné) et 45 g de silice colloïdale vendue sous la dénomination commerciale Klebosol®30 (contenant 30% en poids de $SiO_2$ et 0,5% de $Na_2O$) avec la quantité d'eau qui permet l'extrusion du mélange. Les extrudés sont séchés, concassés de manière à récupérer des grains dont le diamètre moyen en nombre est égal à 0,5 mm, puis calcinés à 550°C sous courant d'azote pendant 2 heures.

200 g d'agglomérés obtenus sont placés dans un réacteur en verre muni d'une double enveloppe régulée à une température de 100°C ± 1°C, puis on ajoute 1,5 L d'une solution aqueuse d'hydroxyde de sodium de concentration 2,5 M et on laisse le milieu réactionnel sous agitation pendant une durée de 4 heures.

On procède ensuite au lavage des agglomérés en 3 opérations successives de lavage à l'eau suivi de la vidange du réacteur. On s'assure de l'efficacité du lavage en mesurant le pH final des eaux de lavage compris entre 10,0 et 10,5.

**Exemple 1** : **Échange cationique au baryum et au potassium**

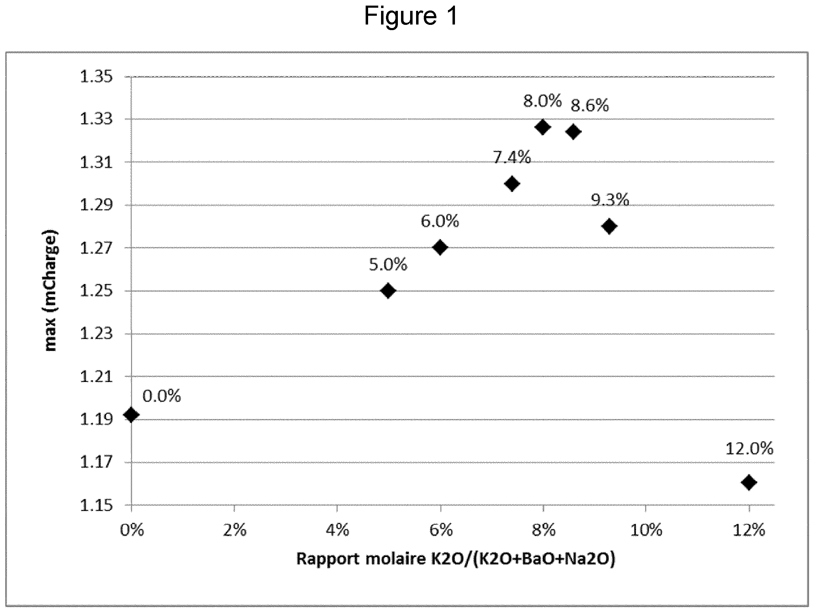

Les cations sodium des agglomérés obtenus sont échangés par des ions baryum et potassium au moyen d'une solution aqueuse à 0,5M de chlorure de potassium et de chlorure de baryum à 95°C en 4 étapes. Les concentrations de chlorure de potassium et de chlorure de baryum dans la solution sont adaptées afin d'atteindre les teneurs en baryum et potassium visées dans l'adsorbant et donc les ratios molaires $K_2O / (K_2O + BaO + Na_2O)$ visés (Figure 1). En particulier, le ratio molaire $K_2O / (K_2O + BaO + Na_2O)$ de 8,0% est atteint avec une solution aqueuse de chlorure de baryum de concentration 0,40 M et de chlorure de potassium de concentration 0,10 M. À chaque étape, le rapport volume de solution sur masse de solide est de 20 mL/g et l'échange est poursuivi pendant 3 heures à chaque fois. Entre chaque échange, le solide est lavé plusieurs fois de manière à le débarrasser des excédents de sel. Les agglomérés sont ensuite séchés à 80°C pendant 2 heures et enfin activés à 250°C pendant 2 heures sous courant d'azote.

La perte au feu mesurée, comme décrit précédemment, est de 5,6% ± 0,1% pour chaque échantillon. Le taux d'échange en baryum+potassium des agglomérés calculés à partir des analyses élémentaires des oxydes de baryum et de sodium par fluorescence X comme décrit dans les techniques de caractérisation est de 99,7 ± 0,2%. En particulier, pour le ratio molaire $K_2O / (K_2O + BaO + Na_2O)$ de 8,0%, la teneur en oxyde de sodium $Na_2O$ est de 0,05% en poids par rapport au poids total de l'adsorbant, la teneur en oxyde de baryum BaO est de 35,15% en poids par rapport au poids total de l'adsorbant, et la teneur en oxyde de potassium $K_2O$ est de 1,89% en poids par rapport au poids total de l'adsorbant.

**Exemple 2** :Test de perçage

Un test de perçage (chromatographie frontale) est ensuite réalisé sur les agglomérés obtenus à l'exemple 1 pour évaluer leur efficacité. La quantité d'adsorbant utilisée pour ce test est d'environ 30 g.

Le mode opératoire pour obtenir les courbes de perçage est le suivant :

- Remplissage de la colonne par le tamis et mise en place dans le banc de test.
- Remplissage par le solvant (toluène) à température ambiante.
- Montée progressive à la température d'adsorption sous flux de solvant (2 cm$^3$/min).
- Injection de solvant à 2 cm$^3$/min lorsque la température d'adsorption est atteinte.
- Permutation solvant/charge pour injecter la charge (2 cm$^3$/min).
- L'injection de la charge est ensuite maintenue un temps suffisant pour atteindre l'équilibre thermodynamique.
- Collecte de la recette du perçage dans un flacon unique puis analyse de la composition de la recette par CPG.

La pression est suffisante pour que la charge reste en phase liquide, soit 1 MPa. La température d'adsorption est de 175°C. La composition de la charge utilisée pour les tests est la suivante :

- *Para-xylène* : 18% en poids
- *Méta-xylène* : 18% en poids
- *Ortho-xylène* : 18% en poids
- *Éthylbenzène* : 18% en poids
- *Para-diéthylbenzène* : 18% en poids
- *Iso-octane* : 10% en poids (celui-ci est utilisé comme traceur pour l'estimation des volumes non-sélectifs et n'intervient pas dans la séparation)

Les sélectivités des composés deux à deux, notées sélectivités binaires $\alpha_{i/k}$ sont calculées à partir des quantités adsorbées $q_i$ et $q_k$ des composés i et k, ces dernières étant déterminées par bilan matière à partir de l'analyse de la composition de la recette du perçage et de la composition de la charge (charge dans laquelle la fraction massique des composés i et k est $y_i$ et $y_k$) :

$$\alpha_{i/k} = \frac{q_i y_k}{q_k y_i}$$

L'évaluation du potentiel de ces adsorbants lors de la mise en oeuvre à contre-courant simulé, est faite en se basant sur la théorie de l'équilibre appliquée aux systèmes multi-constituants à sélectivités constantes telle que décrite par Mazotti, Storti et Morbidelli dans Robust Design of Countercurrent Adsorption Séparation Processes: 2. Multicomponent Systems, AlChE Journal November 1994 Vol. 40, No. 11. En particulier, on se réfère ici à l'équation 8, qui décrit les conditions à satisfaire sur les débits réduits $m_j$ des 4 sections (j=1 à 4) d'une unité de séparation à contre-courant telle que schématisée en Figure 1 de l'article cité pour obtenir une séparation complète.

$$\text{Section 1 : } K_{ss} < m_1 \delta_1 < +\infty$$

$$\text{Section 2 : } K_{wk} < m_2 \delta_2 < K_{sk}$$

$$\text{Section 3 : } K_{wk} < m_3 \delta_3 < K_{sk} \qquad (8)$$

$$\text{Section 4 : } -\frac{\in_p \delta_4}{\sigma(1-\in_p)} < m_4 \delta_4 < K_{ww}$$

Cette équation 8 fait référence aux adsorptivités $K_i$ des différents constituants, ainsi qu'au paramètre $\delta_j$ de chaque section j défini par l'équation 7 :

$$\delta_j = \sum_{l=1}^{NC} K_l y_l^j \qquad (j = 1,...,4) \qquad (7)$$

Il faut noter ici que par définition la sélectivité binaire $\alpha_{i/k}$ entre les composés i et k est égal au rapport des adsorptivités $K_i / K_k$.

Le débit réduit de chaque section de l'unité est défini comme étant le rapport du débit de la phase liquide sur le débit de la phase adsorbée. L'équation 8 indique quels sont les débits réduits limites pour chaque section. Dans une unité de séparation à contre-courant à 4 sections, le débit de charge correspond à la différence entre le débit en zone 3 et le débit en zone 2.

Par conséquent, lorsque l'on veut évaluer la productivité maximale qui peut être atteinte avec un adsorbant donné, on cherche à évaluer la quantité maximale de charge que l'on pourra traiter, c'est-à-dire à évaluer la différence entre le débit maximal en zone 3 et le débit minimal en zone 2. On pourra comparer les performances en terme de productivité maximale de deux adsorbants en comparant leur débit réduit maximal de charge déterminé à partir des débits réduits des zones 2 et 3, respectivement $m_2$ et $m_3$, selon la relation : $max(m_{Charge}) = max(m_3) - min(m_2)$.

Si on considère un système à sélectivités constantes, la composition de la phase liquide qui donne la contrainte la plus forte en zone 2 et en zone 3 est la composition de la phase liquide au point d'injection de la charge dans l'unité. En effet, à partir de ce point la concentration en para-xylène, qui est le composé le plus adsorbé, augmente dans le sens de circulation du solide en zone 2, et diminue dans le sens de circulation du liquide en zone 3. On peut approximer la composition de ce point à la composition de la charge à traiter, et c'est cette composition qui sera utilisée pour évaluer le terme $\delta_2$ et $\delta_3$ de l'équation 8. Les termes $\delta_2$ et $\delta_3$ étant définis par l'équation 7 mentionnée ci-dessus. Pour chaque adsorbant, ce débit réduit $max(m_{Charge})$ est calculé à partir des valeurs de sélectivités binaires mesurées expérimentalement et représenté dans la figure 1 en fonction du ratio molaire $K_2O / (K_2O + BaO + Na_2O)$ (Figure 1). On s'aperçoit que le débit réduit $max(m_{Charge})$ est amélioré pour un ratio molaire $K_2O / (K_2O + BaO + Na_2O)$ compris entre 8,0% et 8,6%.

**Revendications**

1. Adsorbant zéolithique comprenant des cristaux de zéolithe X et comprenant du baryum, du potassium et du sodium, dans lequel la teneur en oxyde de sodium $Na_2O$ est inférieure à 0,3% en poids par rapport la masse totale de l'adsorbant et dans lequel le ratio molaire $K_2O / (K_2O + BaO + Na_2O)$ est compris entre 8,0% et 8,6%, l'incertitude de mesure sur ledit ratio molaire étant de 0,2%.

2. Adsorbant selon la revendication 1, comprenant en outre une phase non zéolithique.

3. Adsorbant selon l'une quelconque des revendications précédentes, dans lequel la teneur totale en oxydes d'ions alcalins ou alcalino-terreux autres que l'oxyde de baryum BaO, l'oxyde de potassium $K_2O$ et l'oxyde de sodium $Na_2O$, est inférieure à 1% en poids par rapport à la masse totale de l'adsorbant.

4. Adsorbant selon l'une quelconque des revendications précédentes, dans lequel les cristaux de zéolithes X ont un rapport atomique Si/Al compris entre 1,00 et 1,50.

5. Adsorbant selon l'une quelconque des revendications précédentes, ayant un diamètre moyen en nombre compris entre 0,2 mm et 2 mm.

6. Adsorbant selon l'une quelconque des revendications précédentes, dans lequel le diamètre moyen en nombre des cristaux de zéolithes X est inférieur ou égal à 1,5 $\mu$m.

7. Adsorbant selon l'une quelconque des revendications précédentes, ayant une perte au feu, mesurée à 950°C selon la norme NF EN 196-2, comprise entre 4,0% et 7,7% en poids.

8. Adsorbant selon l'une quelconque des revendications précédentes, dans lequel la fraction massique de zéolithe X est d'au moins 80% en poids par rapport au poids total de l'adsorbant.

9. Procédé de préparation d'un adsorbant selon l'une quelconque des revendications précédentes, comprenant au moins les étapes de :

   a) agglomération de cristaux de zéolithe X avec un liant, et mise en forme d'aggloméré, puis séchage et calcination,
   b) zéolithisation éventuelle du liant,
   c) échange cationique de l'aggloméré par mise en contact avec une solution d'ions baryum, ou d'ions potassium, ou d'ions baryum et d'ions potassium, à des concentrations comprises entre 0,05 M et 1,5 M, de préférence comprises entre 0,1 M et 1,2 M,
   d) échange cationique au potassium lorsque l'étape c) d'échange est réalisée avec une solution d'ions baryum seuls, ou échange cationique au baryum lorsque l'étape c) d'échange est réalisée avec une solution d'ions potassium seuls,
   e) puis lavage et séchage du produit ainsi traité, et
   f) activation de l'adsorbant zéolithique ainsi obtenu.

10. Procédé selon la revendication 9, dans lequel le liant mis en œuvre dans l'étape a) contient au moins 80 % en poids d'argile zéolithisable et une source de silice, et en ce que le procédé comprend une étape b) de zéolithisation dudit liant zéolithisable par action d'une solution basique alcaline, de préférence avec une solution de concentration comprise entre 0,5 M et 5 M et pendant une durée comprise entre quelques dizaines de minutes et quelques heures.

11. Procédé selon la revendication 9 ou la revendication 10, dans lequel la zéolithe X utilisée à l'étape a) comprend, de préférence est, une zéolithe FAU de type X à porosité hiérarchisée, possédant à la fois des micropores et des mésopores.

12. Utilisation d'un adsorbant selon l'une quelconque des revendications 1 à 8, dans les procédés de :

- séparation de coupes d'isomères aromatiques en C8 et notamment des xylènes,
- séparation d'isomères de toluène substitué tels que nitrotoluène, diéthyltoluène, toluènediamine,
- séparation des crésols,
- séparation des alcools polyhydriques.

13. Utilisation selon la revendication 12, pour la séparation de *para*-xylène à partir de coupes d'isomères aromatiques à 8 atomes de carbone.

14. Procédé de récupération de *para*-xylène à partir de coupes d'isomères d'hydrocarbures aromatiques contenant 8 atomes de carbone, en phase liquide, par adsorption du *para*-xylène au moyen d'un adsorbant selon l'une quelconque des revendications 1 à 8 en présence d'un désorbant, de préférence choisi parmi le toluène et le *para*-diéthylbenzène.

15. Procédé de récupération de *para*-xylène selon la revendication 14 de type lit mobile simulé, de préférence à contre-courant simulé.

16. Procédé de récupération de *para*-xylène à partir de coupes d'isomères d'hydrocarbures aromatiques contenant 8 atomes de carbone, en phase gazeuse, par adsorption du *para*-xylène au moyen d'un adsorbant selon l'une quelconque des revendications 1 à 8 en présence d'un désorbant, de préférence choisi parmi le toluène et le *para*-diéthylbenzène.

**Patentansprüche**

1. Zeolithisches Adsorptionsmittel, umfassend Zeolith-X-Kristalle und umfassend Barium, Kalium und Natrium, wobei der Gehalt an Natriumoxid $Na_2O$ unter 0,3 Gew.-% im Verhältnis zur Gesamtmasse des Adsorptionsmittels liegt und wobei das molare Verhältnis $K_2O$ / ($K_2O$ + BaO + $Na_2O$) zwischen 8,0 % und 8,6 % liegt, wobei die Messunsicherheit beim molaren Verhältnis 0,2 % beträgt.

2. Adsorptionsmittel nach Anspruch 1, umfassend ferner eine nicht-zeolithische Phase.

3. Adsorptionsmittel nach einem der vorangehenden Ansprüche, wobei der Gehalt insgesamt an Oxiden alkalischer oder erdalkalischer Ionen, die andere als Bariumoxid BaO, Kaliumoxid $K_2O$ und Natriumoxid $Na_2O$ sind, kleiner als 1 Gew.-% im Verhältnis zur Gesamtmasse des Adsorptionsmittels ist.

4. Adsorptionsmittel nach einem der vorangehenden Ansprüche, wobei die Zeolith-X-Kristalle ein Atomverhältnis Si/Al haben, das zwischen 1,00 und 1,50 liegt.

5. Adsorptionsmittel nach einem der vorangehenden Ansprüche, das einen zahlenmittleren Durchmesser hat, der zwischen 0,2 mm und 2 mm liegt.

6. Adsorptionsmittel nach einem der vorangehenden Ansprüche, wobei der zahlenmittlere Durchmesser der Zeolith-X-Kristalle kleiner oder gleich 1,5 $\mu$m ist.

7. Adsorptionsmittel nach einem der vorangehenden Ansprüche, das einen Glühverlust, gemessen bei 950 °C gemäß der Norm NF EN 196-2, hat, der zwischen zwischen 4,0 und 7,7 Gew.-% liegt.

8. Adsorptionsmittel nach einem der vorangehenden Ansprüche, wobei der Massenanteil Zeolith X mindestens 80 Gew.-% im Verhältnis zum Gesamtgewicht des Adsorptionsmittels beträgt.

9. Verfahren zur Herstellung eines Adsorptionsmittels nach einem der vorangehenden Ansprüche, umfassend mindestens die folgenden Schritte:

a) Agglomerieren von Zeolith-X-Kristallen mit einem Bindemittel und Versetzen in Agglomeratform, dann Trocknen und Calcinieren,
b) eventuelles Zeolithisieren des Bindemittels,
c) Kationenaustausch des Agglomerats durch Inkontaktversetzen mit einer Lösung aus Bariumionen oder Kaliumionen oder Bariumionen und Kaliumionen in Konzentrationen zwischen 0,05 M und 1,5 M, vorzugsweise zwischen 0,1 M und 1,2 M,
d) Kalium-Kationenaustausch, wenn der Austauschschritt c) mit einer Lösung nur aus Bariumionen durchgeführt wird, oder Barium-Kationenaustausch, wenn der Austauschschritt c) mit einer Lösung nur als Kaliumionen durchgeführt wird,
e) dann Waschen und Trocknen des derart behandelten Produkts, und
f) Aktivieren des derart erhaltenen zeolithischen Adsorptionsmittels.

10. Verfahren nach Anspruch 9, wobei das in Schritt a) verwendete Bindemittel mindestens 80 Gew.-% zeolithisierbaren Ton und eine Siliciumoxidquelle enthält und das Verfahren einen Zeolithisierungsschritt b) des zeolithisierbaren Bindemittels durch Wirken einer alkalischen basischen Lösung, vorzugsweise mit einer Lösung in einer Konzentration zwischen 0,5 M und 5 M und während einer Dauer umfasst, die zwischen einigen Dutzend Minuten und einigen Stunden liegt.

11. Verfahren nach Anspruch 9 oder Anspruch 10, wobei der in Schritt a) verwendete Zeolith X umfasst, vorzugsweise ist, ein FAU-Zeolith vom Typ X mit hierarchisierter Porosität, die gleichzeitig Mikroporen und Mesoporen besitzt.

12. Verwendung eines Adsorptionsmittels nach einem der Ansprüche 1 bis 8 in den folgenden Verfahren:

• Separieren von Schnitten aromatischer C8-Isomere, vor allem der Xylene,
• Separieren von Isomeren substituierten Tuolens wie Nitrotoluen, Diethyltoluen, Toluendiamin,
• Separieren der Cresole,
• Separieren der mehrwertigen Alkohole.

13. Verwendung nach Anspruch 12 für das Separieren von *para*-Xylen aus Schnitten aromatischer Isomere mit 8 Kohlenstoffatomen.

14. Verfahren zur Rückgewinnung von *para*-Xylen aus Schnitten von Isomeren aromatischer Kohlenwasserstoffe mit 8 Kohlenstoffatomen in flüssiger Phase durch Adsorption des *para*-Xylens mittels eines Adsorptionsmittels nach einem der Ansprüche 1 bis 8 bei Anwesenheit eines Desorptionsmittels, vorzugsweise ausgewählt aus dem Toluen und dem para-Diethylbenzol.

15. Verfahren zur Rückgewinnung von *para*-Xylen nach Anspruch 14 vom Typ simuliertes bewegliches Bett, vorzugsweise mit simuliertem Gegenstrom.

16. Verfahren zur Rückgewinnung von *para*-Xylen aus Schnitten von Isomeren aromatischer Kohlenwasserstoffe mit 8 Kohlenstoffatomen in gasförmiger Phase durch Adsorption des *para*-Xylens mittels eines Adsorptionsmittels nach einem der Ansprüche 1 bis 8 bei Anwesenheit eines Desorptionsmittels, vorzugsweise ausgewählt aus dem Toluen und dem para-Diethylbenzol.

## Claims

1. A zeolite adsorbent comprising zeolite X crystals and comprising barium, potassium and sodium, wherein the content of sodium oxide $Na_2O$ is less than 0.3% by weight relative to the total mass of the adsorbent, and wherein the $K_2O$ / ($K_2O$ + $BaO$ + $Na_2O$) mole ratio is comprised between 8.0% and 8.6%, the measuring uncertainty regarding said molar ratio being of 0.2 %.

2. The adsorbent according to claim 1, also comprising a non-zeolite phase.

3. The adsorbent according to any one of the preceding claims, wherein the total content of alkali metal or alkaline-earth metal ion oxides other than barium oxide BaO, potassium oxide $K_2O$ and sodium oxide $Na_2O$ is less than 1% by weight relative to the total mass of the adsorbent.

4. The adsorbent according to any one of the preceding claims, wherein the zeolite X crystals have an Si/Al atomic ratio of between 1.00 and 1.50.

5. The adsorbent according to any one of the preceding claims, having a number mean diameter comprised between 0.2 mm and 2 mm.

6. The adsorbent according to any one of the preceding claims, wherein the number mean diameter of the zeolite X crystals is less than or equal to 1.5 $\mu$m.

7. The adsorbent according to any one of the preceding claims, having a loss on ignition, measured at 950°C according to standard NF EN 196-2, comprised between 4.0% and 7.7% by weight.

8. The adsorbent according to any one of the preceding claims, wherein the mass fraction of zeolite X is at least 80% by weight relative to the total weight of the adsorbent.

9. A process for preparing an adsorbent according to any one of the preceding claims, comprising at least the steps of:

   a) agglomerating zeolite X crystals with a binder, and forming agglomerate, followed by drying and calcination,
   b) optional zeolitization of the binder,
   c) cation exchange of the agglomerate by placing it in contact with a solution of barium ions, or of potassium ions, or of barium ions and potassium ions, at concentrations comprised between 0.05 M and 1.5 M, preferably comprised between 0.1 M and 1.2 M,
   d) cation exchange with potassium when the exchange step c) is performed with a solution of barium ions alone, or cation exchange with barium when the exchange step c) is performed with a solution of potassium ions alone,
   e) followed by washing and drying of the product thus treated, and
   f) activation of the zeolite adsorbent thus obtained.

10. The process according to claim 9, wherein the binder used in step a) contains at least 80 % by weight of zeolitizable clay and a source of silica, and in that the process comprises a step b) of zeolitization of said zeolitizable binder via the action of an alkaline basic solution, preferably a solution of a concentration comprised between 0.5 M and 5 M and for a duration comprised between a few tens of minutes and a few hours.

11. The process according to claim 9 or 10, wherein the zeolite X used in step a) comprises a hierarchically porous type X FAU zeolite, simultaneously having micropores and mesopores.

12. Use of an adsorbent according to any one of the preceding claims in processes for:

   • separating aromatic C8 isomer fractions and especially xylenes,
   • separating substituted toluene isomers such as nitrotoluene, diethyltoluene, toluenediamine,
   • separating cresols,
   • separating polyhydric alcohols.

13. The use according to claim 12, for separating para-xylene from aromatic isomer fractions containing 8 carbon atoms.

14. A process for recovering *para*-xylene from isomer fractions of aromatic hydrocarbons containing 8 carbon atoms, in the liquid phase, by adsorption of the *para*-xylene using an adsorbent according to any one of claims 1 to 8, in the presence of a desorbent, preferably chosen from toluene and para-diethylbenzene.

15. The process for recovering *para*-xylene according to claim 14 of simulated moving bed type, preferably of simulated counter-current type.

16. A process for recovering *para*-xylene from isomer fractions of aromatic hydrocarbons containing 8 carbon atoms, in the gaseous phase, by adsorption of the *para*-xylene using an adsorbent according to any one of claims 1 to 8 in the presence of a desorbent, preferably chosen from toluene and *para*-diethylbenzene.

## Figure 1

EP 3 319 723 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3558730 A
- US 3558732 A
- US 3626020 A
- US 3663638 A
- US 3960774 A
- US 2985589 A
- FR 2903978
- US 8283274 B
- US 8557028 B
- CN 1267185

- US 20150105600 A
- FR 2789914
- US 7785563 B
- WO 2013106816 A
- US 5284992 A
- US 5629467 A
- US 4402832 A
- US 4498991 A
- FR 2999098

**Littérature non-brevet citée dans la description**

- Principles of Adsorption and Adsorption Processes. **RUTHVEN.** Principes de l'Adsorption et des Procédés d'Adsorption. John Wiley & Sons, 1984, 326-407
- **RUTHVEN.** PRINCIPES DE L ADSORPTION ET DES PROCEDES D ADSORPTION. 243
- **INAYAT.** *Angew. Chem. Int. Ed.,* 2012, vol. 51, 1962-1965
- **D. VERBOEKEND.** *Adv. Funct. Mater.,* 2012, vol. 22, 916-928

- **D.W. BRECK.** Zeolite Molecular Sieves. John Wiley and Sons, 1973, 314-315
- **RUTHVEN.** Principles of Adsorption and Adsorption Processes. John Wiley & Sons, 1984
- **MAZOTTI, STORTI.** Morbidelli dans Robust Design of Countercurrent Adsorption Séparation Processes: 2. Multicomponent Systems. *AlChE Journal,* Novembre 1994, vol. 40 (11